# FASCICULE DE BREVET EUROPEEN

(11) **EP 3 215 028 B1**
(45) Date de publication et mention de la délivrance du brevet: **26.09.2018**
(21) Numéro de dépôt: 15798231.5
(22) Date de dépôt: 06.11.2015
(51) Int. Cl.: A61B 17/00, B21D 35/00, A61B 17/16

(54) **PROCEDE DE FABRICATION D'UN ALESOIR**
VERFAHREN ZUR HERSTELLUNG EINER REIBAHLE
METHOD FOR PRODUCING A REAMER

(30) Priorité: 07.11.2014 FR 1460811
(43) Date de publication de la demande: 13.09.2017
(73) Titulaire: Deuxventorio Sarl, 1196 Gland (CH)
(72) Inventeur: GRADEL, Thomas, F-74970 Marignier (FR)
(74) Mandataire: Cabinet Poncet
(86) Numéro de dépôt international: PCT/IB2015/058569
(87) Numéro de publication internationale: WO 2016/071867

(56) Documents cités:
- EP-A1- 2 478 852
- US-A1- 2009 163 921

## Description

### DOMAINE TECHNIQUE DE L'INVENTION

La présente invention concerne un procédé de fabrication d'un alésoir, tel qu'une fraise destinée au fraisage de la cavité acétabulaire d'un patient lors d'une opération chirurgicale de la hanche.

Un exemple d'alésoir connu est décrit dans le document EP 2 359 755. Cet alésoir comprend une base rigide au moins partiellement circulaire à surface latérale sensiblement cylindrique ayant une interface d'accouplement à un porte-outil et des ergots radiaux de réception pour une réception d'un corps de coupe. Sur la base rigide est rapporté et fixé un corps de coupe à paroi fine, perforé et denté, ayant des interfaces d'engagement pour un engagement avec les ergots radiaux de réception de la base rigide.

La base rigide est complexe à fabriquer, notamment pour la réalisation de l'interface d'accouplement et des ergots radiaux de réception. Cette complexité rend le coût de fabrication de l'alésoir très élevé.

Or, pour garantir une stérilité irréprochable au bloc chirurgical et limiter au maximum le risque infectieux pour le patient, de plus en plus de praticiens souhaitent disposer d'un porte-outil à usage unique qui est fourni à l'état stérile au bloc opératoire et qui est jeté après utilisation. Cela oblige à fournir un alésoir peu onéreux, ce qui est impossible dans le cas de l'alésoir du document EP 2 359 755 du fait de la complexité de la base rigide.

Le document US 2009/0163921 décrit un procédé de fabrication d'un alésoir au cours duquel un corps de coupe est fabriqué par découpe et emboutissage. La base rigide présente une forme qu'il n'est pas possible d'obtenir au moyen d'un procédé simple, rapide et peu onéreux. Le document US 2009/0163921 enseigne plus particulièrement de fabriquer la base rigide par un procédé de moulage.

### EXPOSE DE L'INVENTION

Le problème proposé par l'invention est de concevoir un procédé de fabrication permettant une fabrication rapide et à moindre coût d'un alésoir, tel qu'une fraise destinée au fraisage de la cavité acétabulaire d'un patient, comprenant une base rigide à interface d'accouplement à un porte-outil, et comprenant un corps de coupe à paroi fine, perforé et denté.

Pour atteindre ces objets ainsi que d'autres, l'invention propose un procédé de fabrication d'un alésoir, tel qu'une fraise destinée au fraisage de la cavité acétabulaire d'un patient, ledit alésoir comprenant une base rigide à interface d'accouplement à un porte-outil, et comprenant un corps de coupe à paroi fine, perforé et denté ;
selon l'invention, la base rigide est fabriquée séparément du corps de coupe, par découpage et emboutissage sous presse d'un premier flan métallique plat.

Un tel procédé de fabrication est rapide, facile à mettre en oeuvre et suffisamment peu onéreux pour que l'alésoir présente un coût de fabrication suffisamment peu élevé et que les utilisateurs puissent ensuite n'en faire qu'un usage unique.

Avantageusement, lors de l'opération d'emboutissage, on peut déformer le premier flan de façon à conférer à des branches de l'interface d'accouplement une section transversale en U ou en V. On obtient ainsi une interface d'accouplement à branches présentant une rigidité satisfaisante, bien qu'ayant été obtenues à partir du découpage d'un flan plat.

De préférence, le corps de coupe peut être fabriqué séparément de la base rigide, par découpage et emboutissage sous presse d'un deuxième flan métallique plat afin de découper le corps de coupe selon son contour, de perforer le corps de coupe et de former les dents du corps de coupe.

On peut ainsi former, de façon simple, rapide et économique, les dents sur le deuxième flan maintenu à plat car non encore conformé en demi-sphère, ce qui est plus simple que lorsque le corps de coupe est déjà solidarisé à la base rigide et donc conformé en demi-sphère.

Avantageusement, le procédé peut comporter les étapes suivantes :
a) fournir une base rigide,
b) fournir une ébauche de corps de coupe constituée par un deuxième flan plat comportant une pluralité de pétales perforés et dentés, s'étendant radialement depuis une zone centrale à laquelle ils se raccordent jusqu'à une extrémité libre à bord d'extrémité, et séparés les uns des autres par des espaces latéraux radiaux,
c) conformer le corps de coupe en demi-sphère,
d) rapporter la base rigide sur le corps de coupe en demi-sphère.

Un tel procédé de fabrication est facile et rapide à mettre en oeuvre, et peut en outre être automatisé. Après découpage et emboutissage dudit deuxième flan (qui peut présenter une épaisseur constante), celui-ci est conformé en demi-sphère en rapprochant les extrémités libres des pétales pour être ensuite fixé à la base rigide. Ce rapprochement est permis par les espaces latéraux radiaux situés entre les pétales, qui tendent à diminuer lors du rapprochement. Le corps de coupe se présente au final sous la forme d'un flan (éventuellement à épaisseur constante) comportant une pluralité de pétales incurvés s'étendant radialement depuis la zone centrale jusqu'à leur extrémité libre à bord d'extrémité.

Avantageusement, on peut prévoir que :
- la base rigide est au moins partiellement circulaire avec une surface latérale sensiblement cylindrique ayant une interface d'accouplement à un porte-outil et des ergots radiaux de réception pour la réception du corps de coupe,
- le corps de coupe à paroi fine, perforé et denté, comporte des interfaces d'engagement pour un engagement avec les ergots radiaux de réception de la base rigide,
- l'interface d'accouplement et les ergots radiaux de réception ont des facettes latérales parallèles à un axe de cylindre de la surface latérale sensiblement cylindrique de la base rigide,
- lors de l'étape d), on fait pénétrer tous les ergots radiaux de réception dans les interfaces d'engagement respectives par un simple mouvement commun de translation.

La forme particulière de l'interface d'accouplement et des ergots radiaux de réception est compatible avec une fabrication de la base rigide par un procédé de découpage et emboutissage sous presse à partir d'un flan plat métallique. Et le mouvement de translation pour l'assemblage du corps de coupe et de la base rigide est simple à réaliser à l'aide d'un automate.

De préférence, l'extrémité libre de chaque pétale peut comporter au moins une interface d'engagement comportant une encoche ménagée dans le bord d'extrémité du pétale et dans laquelle s'engage l'un des ergots radiaux de réception de la base rigide. Les encoches permettent de bien positionner le flan conformé en demi-sphère par rapport à la base rigide avant que celui-ci ne soit définitivement solidarisé avec la base rigide. On limite ainsi efficacement les risques que le corps de coupe de l'alésoir présente, après assemblage avec la base rigide, une géométrie défectueuse, c'est-à-dire s'écartant de la forme générale d'un hémisphère. Etant ménagées dans le bord d'extrémité des pétales, les encoches peuvent recevoir les ergots radiaux de réception de la base rigide selon un simple mouvement de translation après que le corps de coupe ait été rigoureusement conformé en demi-sphère.

Avantageusement, on peut prévoir que :
- chaque pétale comporte deux bords latéraux s'étendant radialement depuis la zone centrale jusqu'au bord d'extrémité,
- chaque pétale comporte une interface d'engagement ayant deux encoches sensiblement en forme de L respectivement ménagées entre le bord d'extrémité et l'un des bords latéraux.

Les encoches en L de deux pétales adjacents forment ainsi, par coopération, des encoches sensiblement en forme de U aptes à recevoir les ergots radiaux de réception de la base rigide. Les ergots radiaux de réception définissent ainsi un écart prédéterminé satisfaisant entre deux pétales adjacents. Et les extrémités libres des pétales peuvent ainsi être fixées à la base rigide au moins aux deux points extrêmes de leur bord d'extrémité, ce qui limite les risques d'une déformation des pétales par voilage lors des opérations de fraisage de la cavité acétabulaire du patient. Une fixation des extrémités libres des pétales à la base rigide selon la totalité de la longueur de leur bord d'extrémité est également possible.

De préférence, lors d'une étape e), l'extrémité libre des pétales peut être fixée par soudure ou par collage sur la surface latérale sensiblement cylindrique de la base rigide. Une telle fixation est rapide et facile à réaliser, notamment par une automatisation.

De préférence, on peut prévoir que :
- la base rigide est au moins partiellement circulaire avec une surface latérale sensiblement cylindrique,
- l'interface d'accouplement comporte au moins deux branches s'étendant depuis le centre de la base rigide et reliées à la surface latérale sensiblement cylindrique.

Ces branches pourront être reçues dans des porte-outils à platine de connexion de type baïonnette comportant des encoches à profil longitudinal sensiblement en forme de L, tels qu'illustrés dans les documents EP 0 704 191, EP 1 129 667 et EP 1 624 814 et qui équipent déjà les blocs opératoires de la majeure partie des hôpitaux. Les praticiens ne sont ainsi pas obligés de procéder à l'achat d'un porte-outil spécifique pour utiliser l'alésoir selon l'invention.

L'alésoir peut néanmoins comporter un nombre différent de branches sur sa base rigide, par exemple trois ou quatre, voire plus.

Avantageusement, l'interface d'accouplement peut se présenter sous la forme d'une croix dont au moins deux branches s'étendent depuis le centre de la base rigide et sont reliées à la surface latérale sensiblement cylindrique. Les deux branches qui ne s'étendent pas jusqu'à la base rigide permettent de maintenir l'alésoir fixe en orientation autour des directions axiales définies par les deux autres branches. Ce maintien s'effectue par coopération avec la platine de connexion du porte-outil, le plus souvent par un simple appui contre la platine de connexion du porte-outil.

### DESCRIPTION SOMMAIRE DES DESSINS

D'autres objets, caractéristiques et avantages de la présente invention ressortiront de la description suivante de modes de réalisation particuliers, faite en relation avec les figures jointes, parmi lesquelles :
- la figure 1 est une vue en perspective d'un mode de réalisation d'un alésoir dont la base rigide au moins est obtenue à l'aide d'un procédé de fabrication selon l'invention ;
- la figure 2 est une vue partielle de détail et en perspective de la base rigide de l'alésoir de la figure 1 ;
- la figure 3 est une vue de dessus de la figure 2 ;
- la figure 4 est une vue en perspective et de dessous de la base rigide de l'alésoir de la figure 1 ;
- la figure 5 est une autre vue en perspective et de dessous de la base rigide de l'alésoir de la figure 1, prise selon une orientation à 90° par rapport à la vue de la figure 4 ;
- la figure 6 est une vue en perspective du corps de coupe de l'alésoir de la figure 1 ;
- la figure 7 est une vue en perspective du corps de coupe de l'alésoir de la figure 1, avant sa conformation en demi-sphère ; et
- la figure 8 est une vue schématique et en perspective illustrant un procédé de fabrication de l'alésoir de la figure 1.

### DESCRIPTION DES MODES DE REALISATION PREFERES

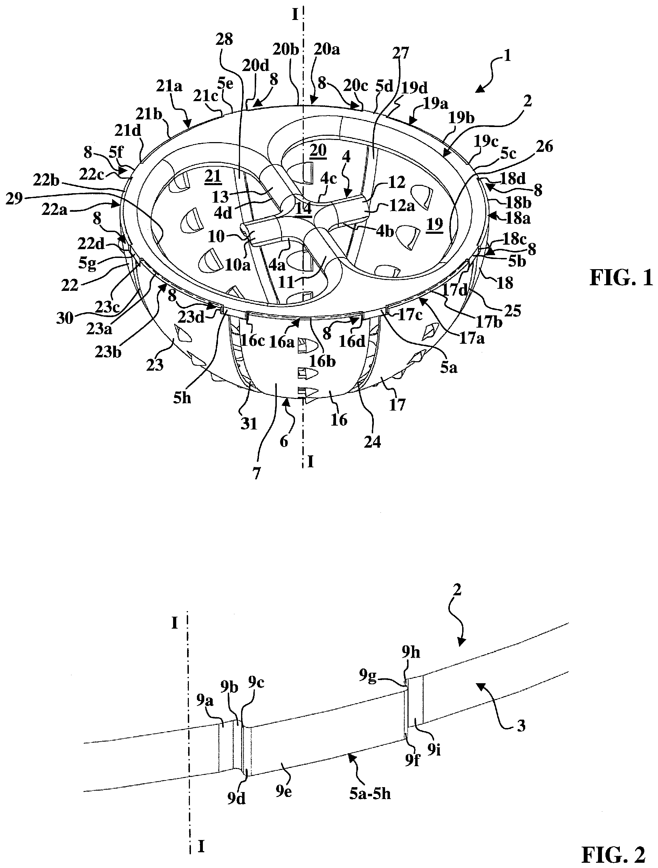

Un exemple d'alésoir 1 selon l'invention, de type fraise chirurgicale destinée au fraisage de la cavité acétabulaire d'un patient, est illustré sur les figures 1 à 7.

Cet alésoir comprend :
- une base rigide 2 au moins partiellement circulaire à surface latérale 3 sensiblement cylindrique ayant une interface d'accouplement 4 à un porte-outil et des ergots radiaux de réception 5a à 5h pour une réception d'un corps de coupe 6,
- un corps de coupe 6 à paroi 7 fine, perforé et denté, ayant des interfaces d'engagement 8 pour un engagement avec les ergots radiaux de réception 5a à 5h de la base rigide 2.

La surface latérale 3 sensiblement cylindrique s'étend selon un axe de cylindre I-I.

On voit plus particulièrement sur les figures 1, 4 et 5 que l'interface d'accouplement 4 a des facettes latérales 4a à 4d parallèles à l'axe de cylindre I-I de la surface latérale 3 sensiblement cylindrique de la base rigide 2. La fabrication de l'interface d'accouplement 4 avec des facettes latérales 4a à 4d orientées de cette façon est possible par un procédé de découpage et emboutissage sous presse.

De même, les ergots radiaux de réception 5a à 5h ont des facettes latérales 9a à 9i parallèles à l'axe de cylindre I-I de la surface latérale 3 sensiblement cylindrique de la base rigide 2. La fabrication des ergots radiaux de réception 5a à 5h avec des facettes latérales 9a à 9i orientées de cette façon est également possible par un procédé de découpage et emboutissage sous presse, de sorte que toute la base rigide 2 en elle-même peut être obtenue par un procédé de découpage et emboutissage sous presse d'un premier flan 33 métallique plat.

Le procédé de découpage et emboutissage sous presse peut être effectué à l'aide d'une presse mécanique de découpe-emboutissage munie d'un outillage progressif (outils à suivre et/ou outils de transfert).

L'interface d'accouplement 4 comporte quatre branches 10 à 13 s'étendant depuis le centre 14 de la base rigide 2. Les deux branches 11 et 13 sont reliées à la surface latérale 3 sensiblement cylindrique. Les deux branches 10 et 12 se terminent par des extrémités libres 10a et 12a. Les branches 10 et 12 peuvent toutefois se prolonger jusqu'à surface latérale 3 sensiblement cylindrique, comme les branches 11 et 13.

L'interface d'accouplement 4 se présente ainsi sous la forme d'une croix dont au moins deux branches 11 et 13 s'étendent depuis le centre 14 de la base rigide 2 et sont reliées à la surface latérale 3 sensiblement cylindrique.

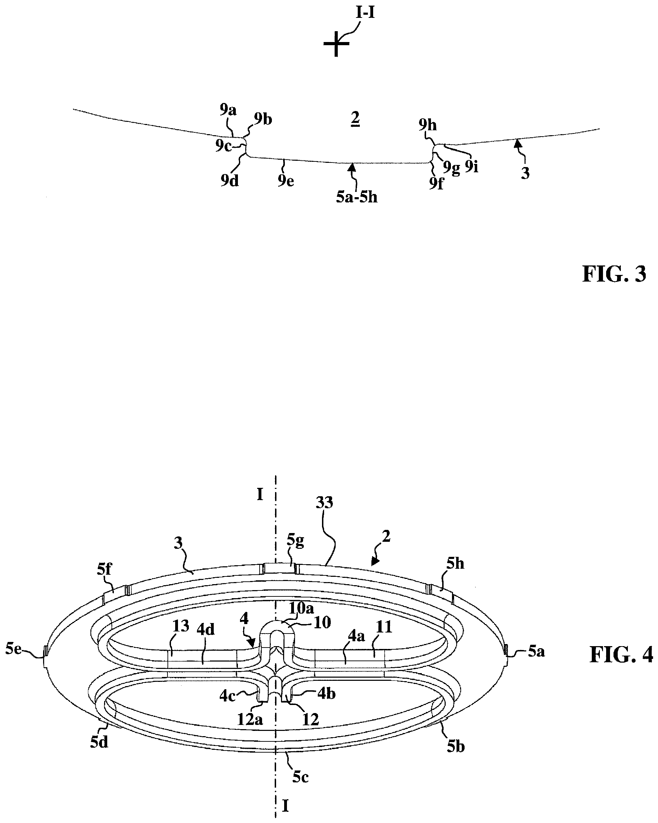

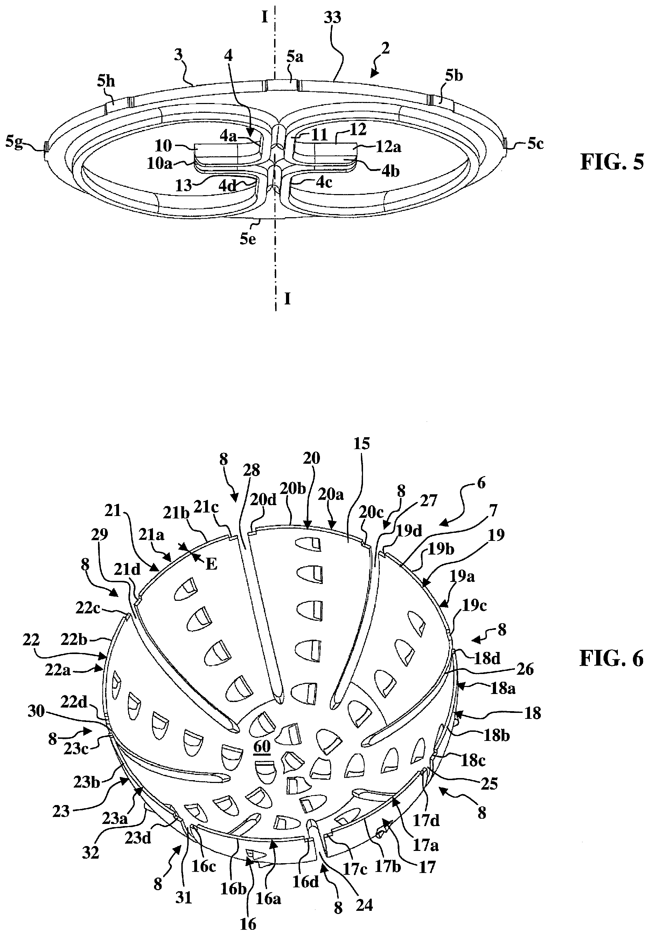

On voit plus particulièrement sur les figures 4 et 5 que les branches 10 à 13 ont une section transversale sensiblement en forme de U. Une conformation ressemblant plus en forme à un V est également possible. Le côté ouvert du U est orienté en direction de l'intérieur du corps de coupe 6 lorsque l'alésoir 1 est assemblé (figure 1). Cette conformation en U des branches 10 à 13 est également réalisable lors du même procédé de découpage et emboutissage sous presse à partir du premier flan 33 métallique plat, et procure aux branches 10 à 13 une bonne rigidité pour transmettre le couple de rotation qui sera communiqué par le porte-outil accroché aux branches 10 à 13, de façon similaire à ce qui est illustré dans les documents EP 0 704 191, EP 1 129 667 et EP 1 624 814.

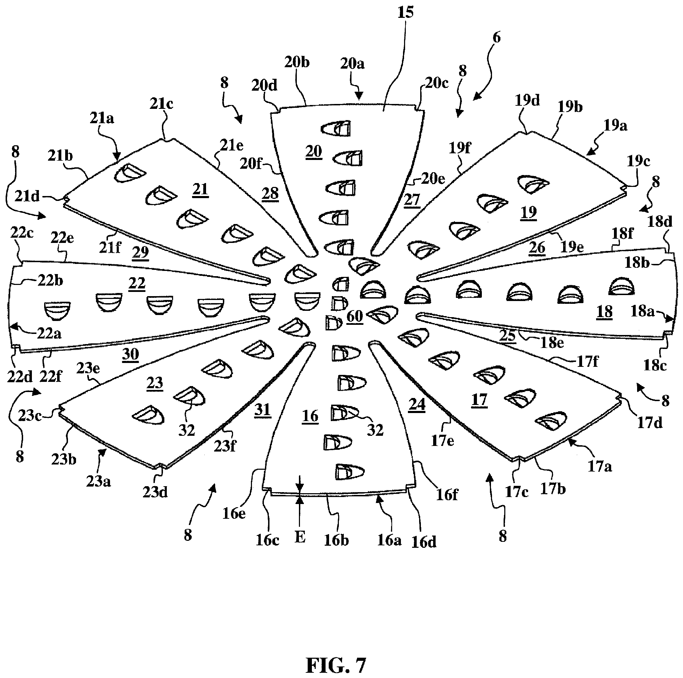

On voit plus particulièrement sur les figures 6 et 7 que le corps de coupe 6 est constitué par un deuxième flan 15 à épaisseur E constante comportant une pluralité de pétales 16 à 23 s'étendant radialement depuis une zone centrale 60 jusqu'à une extrémité libre 16a à 23a à bord d'extrémité 16b à 23b, et séparés les uns des autres par des espaces latéraux radiaux 24 à 31.

Sur la figure 7, le deuxième flan 15 est illustré juste après sa fabrication, réalisée séparément de la fabrication de la base rigide 2, par un procédé de découpage et emboutissage sous presse pour découper le corps de coupe 6, perforer le corps de coupe 6 et former les dents 32 du corps de coupe 6. Le procédé de découpage et emboutissage sous presse peut également être effectué à l'aide d'une presse mécanique de découpe-emboutissage munie d'un outillage progressif (outils à suivre et/ou outils de transfert).

Sur la figure 6, le deuxième flan 15 du corps de coupe 6 est illustré juste après sa conformation en demi-sphère. Cette conformation est réalisée préalablement à la solidarisation du corps de coupe 6 à la base rigide 2.

On voit plus particulièrement sur la figure 7 que chaque pétale 16 à 23 comporte au moins une interface d'engagement 8 ayant deux encoches en L 16c à 23c et 16d à 23d ménagées dans le bord d'extrémité 16b à 23b et les bords latéraux 16e à 23e et 16f à 23f des pétales 16 à 23.

Les encoches en L 16c à 23c et 16d à 23d forment deux à deux des encoches sensiblement en forme de U dans lesquelles sont reçus les ergots radiaux de réception 5a à 5h de la base rigide 2.

Les encoches sont ainsi orientées, après conformation en demi-sphère du deuxième flan 15, de façon à recevoir les ergots radiaux de réception 5a à 5h selon un simple mouvement de translation selon la direction définie par l'axe de cylindre 1-1 de la surface latérale 3 sensiblement cylindrique de la base rigide 2.

En alternative, des encoches sensiblement en forme de U peuvent être réalisées sensiblement au milieu des bords d'extrémité 16b à 23b.

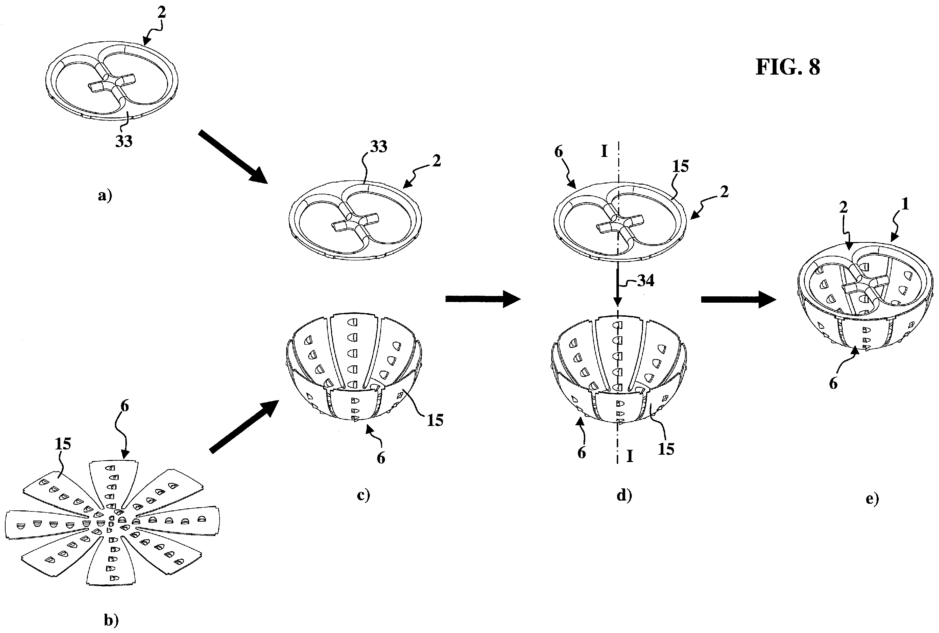

Le procédé de fabrication de l'alésoir 1 va désormais être expliqué, notamment à l'aide de la figure 8.

Lors d'une étape a), on fournit une base rigide 2 (la base rigide 2 est identique à celle des figures 4 et 5). Cette base rigide 2 est obtenue à partir du découpage et emboutissage sous presse d'un premier flan 33.

Lors d'une étape b), on fournit une ébauche de corps de coupe 6 constituée par un deuxième flan plat 15 comportant une pluralité de pétales 16 à 23 perforés et dentés s'étendant radialement depuis une zone centrale 60 à laquelle ils se raccordent jusqu'à une extrémité libre 16a à 23a à bord d'extrémité 16b à 23b. Les pétales 16 à 23 sont séparés les uns des autres par des espaces latéraux radiaux 24 à 31. Le deuxième flan 15 se présente à plat. Le découpage selon sa périphérie de l'ébauche de corps de coupe 6, la perforation des pétales 16 à 23 et la formation des dents 32 sont réalisés par découpage et emboutissage sous presse. On obtient ainsi le corps de coupe 6 « à plat » tel qu'illustré sur la figure 7.

Lors d'une étape c), on conforme le corps de coupe 6 en demi-sphère, par exemple en poussant le deuxième flan 15 dans un empreinte creuse hémisphérique, par exemple au moyen d'un poussoir de forme hémisphérique complémentaire à l'empreinte creuse hémisphérique. On obtient ainsi le corps de coupe 6 « conformé » tel qu'illustré sur la figure 6.

Lors d'une étape d), on rapporte la base rigide 2 sur le corps de coupe 6 en demi-sphère en faisant pénétrer les ergots radiaux de réception 5a à 5h dans les interfaces d'engagement 8. Du fait de l'orientation des encoches 16c à 23c et 16d à 23d, les ergots radiaux de réception 5a à 5h s'engagent dans les interfaces d'engagement 8 par un simple mouvement de translation selon l'axe de cylindre I-I, illustré par la flèche 34.

Puis, lors d'une étape e), on fixe par soudure ou par collage les extrémités libres 16a à 23a des pétales 16 à 23 sur la surface latérale 3 sensiblement cylindrique de la base rigide 2. On obtient alors l'alésoir tel qu'illustré sur la figure 1.

Cette fixation peut se faire par des points de collage ou de soudure effectués de façon discrète au niveau des encoches en L 16c à 23c et 16d à 23d, c'est-à-dire aux deux points extrêmes des bords d'extrémité 16b à 23b des pétales 16 à 23. Cela limite les risques d'une déformation par torsion et voilage des pétales 16 à 23 lors des opérations ultérieures de fraisage de la cavité acétabulaire du patient. Une fixation des extrémités libres 16a à 23a des pétales 16 à 23 à la base rigide 2 selon la totalité de la longueur de leur bord d'extrémité 16b à 23b est également possible au moyen d'un cordon de colle ou de soudure continus.

La présente invention n'est pas limitée aux modes de réalisation qui ont été explicitement décrits, mais elle en inclut les diverses variantes et généralisations contenues dans le domaine des revendications ci-après.

## Revendications

1. Procédé de fabrication d'un alésoir (1) tel qu'une fraise destinée au fraisage de la cavité acétabulaire d'un patient, ledit alésoir (1) comprenant une base rigide (2) à interface d'accouplement (4) à un porte-outil, et comprenant un corps de coupe (6) à paroi (7) fine, perforé et denté, **caractérisé en ce que** la base rigide (2) est fabriquée séparément du corps de coupe (6), par découpage et emboutissage sous presse d'un premier flan (33) métallique plat.

2. Procédé de fabrication selon la revendication 1, **caractérisé en ce que** lors de l'opération d'emboutissage, on déforme le premier flan (33) de façon à conférer à des branches (10-13) de l'interface d'accouplement (4) une section transversale en U ou en V.

3. Procédé de fabrication selon l'une des revendications 1 ou 2, **caractérisé en ce que** le corps de coupe (6) est fabriqué séparément de la base rigide (2), par découpage et emboutissage sous presse d'un deuxième flan (15) métallique plat afin de découper le corps de coupe (6) selon son contour, de perforer le corps de coupe (6) et de former les dents (32) du corps de coupe (6).

4. Procédé de fabrication selon l'une des revendications 2 ou 3, **caractérisé en ce qu'**il comporte les étapes suivantes :
a) fournir une base rigide (2),
b) fournir une ébauche de corps de coupe (6) constituée par un deuxième flan plat (15) comportant une pluralité de pétales (16-23) perforés et dentés, s'étendant radialement depuis une zone centrale (60) à laquelle ils se raccordent jusqu'à une extrémité libre (16a-23a) à bord d'extrémité (16b-23b), et séparés les uns des autres par des espaces latéraux radiaux (24-31),
c) conformer le corps de coupe (6) en demi-sphère,
d) rapporter la base rigide (2) sur le corps de coupe (6) en demi-sphère.

5. Procédé de fabrication selon la revendication 4, **caractérisé en ce que** :
- la base rigide (2) est au moins partiellement circulaire avec une surface latérale (3) sensiblement cylindrique ayant une interface d'accouplement (4) à un porte-outil et des ergots radiaux de réception (5a-5h) pour la réception du corps de coupe (6),
- le corps de coupe (6) à paroi (7) fine, perforé et denté, comporte des interfaces d'engagement (8) pour un engagement avec les ergots radiaux de réception (5a-5h) de la base rigide (2),
- l'interface d'accouplement (4) et les ergots radiaux de réception (5a-5h) ont des facettes latérales (4a-4d ; 9a-9i) parallèles à un axe de cylindre (I-I) de la surface latérale (3) sensiblement cylindrique de la base rigide (2),
et **en ce que**, lors de l'étape d), on fait pénétrer tous les ergots radiaux de réception (5a-5h) dans les interfaces d'engagement (8) respectives par un simple mouvement commun de translation (34).

6. Procédé de fabrication selon la revendication 5, **caractérisé en ce que** l'extrémité libre (16a-23a) de chaque pétale (16-23) comporte au moins une interface d'engagement (8) comportant une encoche (16c-23c ; 16d-23d) ménagée dans le bord d'extrémité (16b-23b) du pétale (16-23) et dans laquelle s'engage l'un des ergots radiaux de réception (5a-5h) de la base rigide (2).

7. Procédé de fabrication selon la revendication 6, **caractérisé en ce que** :
- chaque pétale (16-23) comporte deux bords latéraux (16e-23e ; 16f-23f) s'étendant radialement depuis la zone centrale (60) jusqu'au bord d'extrémité (16b-23b),
- chaque pétale (16-23) comporte une interface d'engagement (8) ayant deux encoches sensiblement en forme de L (16c-23c; 16d-23d) respectivement ménagées entre le bord d'extrémité (16b-23b) et l'un des bords latéraux (16e-23e ; 16f-23f).

8. Procédé de fabrication selon l'une quelconque des revendications 4 à 7, **caractérisé en ce que**, lors d'une étape ultérieure e), on fixe par soudure ou par collage les extrémités libres (16a-23a) des pétales (16-23) sur une surface latérale (3) sensiblement cylindrique de la base rigide (2).

9. Procédé de fabrication selon l'une quelconque des revendications 1 à 8, **caractérisé en ce que** :
- la base rigide (2) est au moins partiellement circulaire avec une surface latérale (3) sensiblement cylindrique,
- l'interface d'accouplement (4) comporte au moins deux branches (10-13) s'étendant depuis le centre (14) de la base rigide (2) et reliées à la surface latérale (3) sensiblement cylindrique.

10. Procédé de fabrication selon la revendication 9, **caractérisé en ce que** l'interface d'accouplement (4) se présente sous la forme d'une croix.

## Patentansprüche

1. Verfahren zur Herstellung einer Reibahle (1), wie einer Fräse, vorgesehen zum Fräsen der Hüftgelenkspfanne eines Patienten, welche Reibahle (1) ein steifes Basiselement (2) mit einer Verbindungsstelle zur Kopplung (4) an einen Werkzeughalter und einen perforierten und bezahnten Schneidkörper (6) mit dünner Wandung (7) umfasst, **dadurch gekennzeichnet, dass** das steife Basiselement (2) separat von dem Schneidkörper (6) durch Zuschneiden und Umformen eines ersten flachen Metallrohlings (33) in einer Presse hergestellt wird.

2. Herstellungsverfahren nach Anspruch 1, **dadurch gekennzeichnet, dass** während des Umformvorgangs der erste Rohling (33) derart verformt wird, dass Abschnitte (10-13) der Verbindungsstelle zur Kopplung (4) mit einem U-förmigen oder einem V-förmigen Querschnitt ausgebildet werden.

3. Herstellungsverfahren nach einem der Ansprüche 1 oder 2, **dadurch gekennzeichnet, dass** der Schneidkörper (6) separat von dem steifen Basiselement (2) durch Schneiden und Umformen eines zweiten flachen Metallrohlings (15) in einer Presse hergestellt wird, um den Schneidkörper (6) gemäß dessen Kontur zuzuschneiden, den Schneidkörper (6) zu perforieren und die Zähne (32) des Schneidkörpers (6) zu formen.

4. Herstellungsverfahren nach einem der Ansprüche 2 oder 3, **dadurch gekennzeichnet, dass** das Verfahren die nachfolgenden Schritte umfasst:
a) Bereitstellen eines steifen Basiselements (2),
b) Bereitstellen einer Ausgangsform eines Schneidkörpers (6), gebildet aus einem zweiten, eine Vielzahl an perforierten und bezahnten Blättern (16-23) umfassenden, flachen Rohling (15), welche sich radial von einem Zentralbereich (60) ausgehend, an welchen sich diese anschließen, bis hin zu einem freien Ende (16a-23a) mit einer Endkante (16b-23b) erstrecken und welche durch lateral radiale Zwischenräume (24-31) voneinander beabstandet sind,
c) Ausformen des Schneidkörpers (6) zu einer Halbkugel,
d) Aufsetzen des steifen Basiselements (2) auf den halbkugelförmigen Schneidkörper (6).

5. Herstellungsverfahren nach Anspruch 4, **dadurch gekennzeichnet, dass**:
- das steife Basiselement (2) zumindest teilweise kreisförmig ist, mit einer im Wesentlichen zylindrischen, lateralen Oberfläche (3), aufweisend eine Verbindungsstelle zur Kupplung (4) an einen Werkzeughalter und radiale Aufnahmevorsprünge (5a-5h) zur Aufnahme des Schneidkörpers (6),
- der perforierte und bezahnte Schneidkörper (6) mit dünner Wandung (7) Verbindungsstellen zum Eingriff (8) umfasst, um einen Eingriff mit den radialen Aufnahmevorsprüngen (5a-5h) des steifen Basiselements (2) auszubilden,
- die Verbindungsstelle zur Kopplung (4) und die radialen Aufnahmevorsprünge (5a-5h) laterale, parallel zu einer Zylinderachse (I-I) der im Wesentlichen zylindrischen, lateralen Oberfläche (3) des steifen Basiselements (2) verlaufende Fasen (4a-4d; 9a-9i) aufweisen,
und dadurch, dass während des Schritts d), alle radialen Aufnahmevorsprünge (5a-5h) mittels einer einfachen gemeinsamen Translationsbewegung (34) in die entsprechenden Verbindungsstellen zum Eingriff (8) eingebracht werden.

6. Herstellungsverfahren nach Anspruch 5, **dadurch gekennzeichnet, dass** das freie Ende (16a-23a) eines jeden Blatts (16-23) mindestens eine Verbindungsstelle zum Eingriff (8) umfasst, welche eine in der Endkante (16b-23b) des Blatts (16-23) ausgesparte Einkerbung (16c-23c; 16d-23d) umfasst und in welche einer der radialen Aufnahmevorsprünge (5a-5h) des steifen Basiselements (2) eingreift.

7. Herstellungsverfahren nach Anspruch 6, **dadurch gekennzeichnet, dass**:
- jedes Blatt (16-23) zwei laterale Kanten (16e-23e; 16f-23f) umfasst, welche sich radial von dem Zentralbereich (60) ausgehend bis hin zu den Endkanten (16b-23b) erstrecken,
- jedes Blatt (16-23) eine Verbindungsstelle zum Eingriff (8) umfasst, aufweisend zwei jeweils zwischen der Endkante (16b-23b) und einer der lateralen Kanten (16e-23e; 16f-23f) ausgesparte Einkerbungen, im Wesentlichen in der Form eines L (16c-23c; 16d-23d).

8. Herstellungsverfahren nach einem der Ansprüche 4 bis 7, **dadurch gekennzeichnet, dass** während eines nachfolgenden Schritts e) die freien Enden (16a-23a) der Blätter (16-23) mittels Schweißen oder mittels Kleben auf einer im Wesentlichen zylindrischen, lateralen Oberfläche (3) des steifen Basiselements (2) fixiert werden.

9. Herstellungsverfahren nach einem der Ansprüche 1 bis 8, **dadurch gekennzeichnet, dass**:
- das steife Basiselement (2) zumindest teilweise kreisförmig ist, mit einer im Wesentlichen zylindrischen, lateralen Oberfläche (3),
- die Verbindungsstelle zur Kopplung (4) mindestens zwei Abschnitte (10-13) umfasst, welche sich ausgehend von einem Zentrum (14) des steifen Basiselements (2) erstrecken und an die im Wesentlichen zylindrische, laterale Oberfläche (3) anbinden.

10. Herstellungsverfahren nach Anspruch 9, **dadurch gekennzeichnet, dass** die Verbindungsstelle zur Kopplung (4) die Form eines Kreuzes aufweist.

## Claims

1. A method for producing a reamer (1) such as a milling cutter intended to mill the acetabular cavity of a patient, said reamer (1) comprising a rigid base (2) with a coupling interface (4) for coupling to a tool holder, and comprising a cutter body (6) with a thin, perforated and toothed wall (7), **characterized in that** the rigid base (2) is produced separately from the cutter body (6), by cutting out and press-stamping of a first flat metal panel (33).

2. The production method as claimed in claim 1, **characterized in that**, during the stamping operation, the first panel (33) is deformed in such a way as to give branches (10-13) of the coupling interface (4) a U-shaped or V-shaped cross section.

3. The production method as claimed in either of claims 1 and 2, **characterized in that** the cutter body (6) is produced separately from the rigid base (2), by cutting out and press-stamping of a second flat metal panel (15) in order to cut out the cutter body (6) along its contour, perforate the cutter body (6) and form the teeth (32) of the cutter body (6).

4. The production method as claimed in either of claims 2 and 3, **characterized in that** it has the following steps:
a) supplying a rigid base (2),
b) supplying a cutter body (6) blank formed by a second flat panel (15) having a plurality of perforated and toothed petals (16-23) which extend radially from a central zone (60), to which they are connected, as far as a free end (16a-23a) with an end edge (16b-23b), and which are separated from one another by radial lateral spaces (24-31),
c) shaping the cutter body (6) into a hemisphere,
d) attaching the rigid base (2) to the hemispherical cutter body (6).

5. The production method as claimed in claim 4, **characterized in that**:
- the rigid base (2) is at least partially circular with a substantially cylindrical lateral surface (3) having a coupling interface (4) for coupling to a tool holder, and having radial receiving lugs (5a-5h) for receiving the cutter body (6),
- the perforated and toothed cutter body (6) with the thin wall (7) has engagement interfaces (8) for engaging with the radial receiving lugs (5a-5h) of the rigid base (2),
- the coupling interface (4) and the radial receiving lugs (5a-5h) have lateral facets (4a-4d; 9a-9i) parallel to a cylinder axis (I-I) of the substantially cylindrical lateral surface (3) of the rigid base (2),
and **in that**, during step d), all the radial receiving lugs (5a-5h) are caused to enter the respective engagement interfaces (8) by a simple combined movement of translation (34).

6. The production method as claimed in claim 5, **characterized in that** the free end (16a-23a) of each petal (16-23) has at least one engagement interface (8) having a notch (16c-23c; 16d-23d) which is formed in the end edge (16b-23b) of the petal (16-23) and in which one of the radial receiving lugs (5a-5h) of the rigid base (2) engages.

7. The production method as claimed in claim 6, **characterized in that**:
- each petal (16-23) has two lateral edges (16e-23e; 16f-23f) extending radially from the central zone (60) as far as the end edge (16b-23b),
- each petal (16-23) has an engagement interface (8) having two substantially L-shaped notches (16c-23c; 16d-23d), respectively, formed between the end edge (16b-23b) and one of the lateral edges (16e-23e; 16f-23f).

8. The production method as claimed in any one of claims 4 through 7, **characterized in that**, during a subsequent step e), the free ends (16a-23a) of the petals (16-23) are fixed by welding or by adhesive bonding to a substantially cylindrical lateral surface (3) of the rigid base (2).

9. The production method as claimed in any one of claims 1 through 8, **characterized in that**:
- the rigid base (2) is at least partially circular with a substantially cylindrical lateral surface (3),
- the coupling interface (4) has at least two branches (10-13) which extend from the center (14) of the rigid base (2) and which are connected to the substantially cylindrical lateral surface (3).

10. The production method as claimed in claim 9, **characterized in that** the coupling interface (4) is in the form of a cross.
